(19) Europäisches Patentamt European Patent Office Office européen des brevets

(11) **EP 4 664 397 A1**

(12) **EUROPEAN PATENT APPLICATION**
published in accordance with Art. 153(4) EPC

(43) Date of publication:
17.12.2025 Bulletin 2025/51

(21) Application number: 24752768.2

(22) Date of filing: 31.01.2024

(51) International Patent Classification (IPC):
G06T 7/00 (2017.01)      G06V 10/774 (2022.01)
G06V 10/82 (2022.01)      G06V 10/80 (2022.01)
G06T 3/40 (2024.01)       G06V 20/40 (2022.01)

(52) Cooperative Patent Classification (CPC):
G06T 3/40; G06T 7/00; G06V 10/774; G06V 10/80;
G06V 10/82; G06V 20/40

(86) International application number:
PCT/CN2024/075039

(87) International publication number:
WO 2024/164912 (15.08.2024 Gazette 2024/33)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 07.02.2023 CN 202310069871

(71) Applicant: Tianjin Yujin Artificial Intelligence
Medical
Technology Co., Ltd
Tianjin 300457 (CN)

(72) Inventors:
• FENG, Yue
Tianjin 300457 (CN)
• MU, Jinbao
Tianjin 300457 (CN)
• ZHENG, Zhongqing
Tianjin 300457 (CN)

(74) Representative: Cabinet Chaillot
16/20, avenue de l'Agent Sarre
B.P. 74
92703 Colombes Cedex (FR)

(54) **ENDOSCOPIC TARGET STRUCTURE EVALUATION SYSTEM AND METHOD, DEVICE, AND STORAGE MEDIUM**

(57) The present invention relates to an endoscopic target structure evaluation system and method, a device, and a storage medium. The endoscopic target structure evaluation system includes: a frozen image detection module, which is used for respectively freezing images of a digestive-tract endoscopic target structure from endoscopic examination images before an endoscope withdrawing operation and endoscopic examination images after the endoscope withdrawing operation to respectively obtain a first frozen image and a second frozen image; and a digestive-tract endoscopic target measurement module, which is used for determining a length of the digestive-tract endoscopic target structure according to a first long-axis distance of the first frozen image, a second long-axis distance of the second frozen image, an endoscope withdrawing distance, a length occupied by a single pixel, and a number of pixel points at the first long-axis distance. According to the present invention, problems existing in a visual inspection method and a comparison measurement method with biopsy forceps are effectively solved, so that increasing for accuracy of estimating a lesion size by a doctor is contributed.

FIG. 1

**Description**

CROSS-REFERENCE DESCRIPTION

The present application claims priority to the Chinese patent application with an application number 202310069871.8 and titled "ENDOSCOPIC TARGET STRUCTURE EVALUATION SYSTEM AND METHOD, DEVICE, AND STORAGE MEDIUM", which is filed with the Patent Office of the People's Republic of China on February 7, 2023, and the entire contents thereof are incorporated in the present invention by reference.

BACKGROUND OF THE INVENTION

1. Technical Field

The present invention relates to the field of artificial intelligence, and particularly relates to an endoscopic target structure evaluation system and method, a device, and a storage medium.

2. Description of Related Art

Digestive-tract endoscopic examination, as a main means for observing a target condition in a digestive tract, and particularly for diagnosis and treatment for lesions of the digestive tract, has been widely applied, however, the digestive-tract endoscopic examination has a high rate of missed diagnosis for some digestive-tract endoscopic targets (such as colorectal polyps and adenomas), and the rate of missed diagnosis may be greatly reduced through assistance of artificial intelligence. After a digestive-tract endoscopic target is found through the digestive-tract endoscopic examination, a doctor needs to observe a shape of the target and measure a size of the target, and the size of the digestive-tract endoscopic target is one of main criteria for risk classification and treatment manner selection for the digestive-tract endoscopic target.

Main methods for estimating the size of the digestive-tract endoscopic target include a visual inspection method and a comparison measurement method with biopsy forceps. The visual inspection method is that a doctor estimates the size of the digestive-tract endoscopic target according to own experience, and is high in subjectivity, and different doctors have large differences in estimating the size of the same digestive-tract endoscopic target due to differences in operation habits and experience of the doctors. The comparison measurement method with biopsy forceps refers to placing endoscopic instruments such as the biopsy forceps in an endoscopic examination process, and comparing with the size of the digestive-tract endoscopic target to achieve a purpose of auxiliary measurement. This method requires placement for the endoscopic instruments in the examination process, and is not applicable for examinational ( nontherapeutic) endoscopic examinations that do not require the placement for the instruments in the examination process; and moreover, placement positions and angles of the instruments cause high errors in measurement for the size of the digestive-tract endoscopic target, so that the comparison measurement method with the biopsy forceps has certain limitations through taking into account the above two points.

Therefore, the operation habits and experience of the doctors in prior art, as well as a shape and an angle of a lens of an endoscope, are all main factors affecting measurement for a lesion size. How to increase accuracy of estimating the lesion size by the doctor and establish a unified standard urgently requires improvement in the prior art.

BRIEF SUMMARY OF THE INVENTION

The example of the present invention provides an endoscopic target structure evaluation system and method, a device, and a storage medium, for at least partially solving the above problems.

In the present invention, the above digestive-tract endoscopic target may be targets in normal physiological states, such as including but not limited to tumors, foreign bodies, blood vessels, and fecal masses; and the above digestive-tract endoscopic target may also be targets in pathological states, such as including but not limited to colorectal polyps and adenomas.

In a first aspect, the present invention provides an endoscopic target structure evaluation system, and the endoscopic target structure evaluation system includes:

a frozen image detection module, which is used for respectively freezing images of a digestive-tract endoscopic target structure from endoscopic examination images before an endoscope withdrawing operation and endoscopic examination images after the endoscope withdrawing operation to respectively obtain a first frozen image and a second frozen image; and

a digestive-tract endoscopic target measurement module, which is used for determining a length of the digestive-tract endoscopic target structure according to a first long-axis distance of the first frozen image, a second long-axis distance of the second frozen image, an endoscope withdrawing distance, a length occupied by a single pixel, and a number of pixel points at the first long-axis distance.

Alternatively, the digestive-tract endoscopic target measurement module determines a length $B$ of the digestive-tract endoscopic target structure through adopting a following formula:

$$B = \frac{B_2 \times L}{B_2 - B_1} \times \vartheta \times Pixs(B_1)$$

where $B_1$ represents a first long-axis distance of the first frozen image, $B_2$ represents a second long-axis distance of the second frozen image, $L$ represents an endoscope withdrawing distance, $\vartheta$ represents a length occupied by a single pixel, and $Pixs(B_1)$ represents a number of pixel points at the first long-axis distance; and

a determination manner for $\vartheta$ is placing a pre-arranged precision caliper on a horizontal table surface, keeping a lens away from the caliper by a distance $L$, taking a photo, measuring a pixel width occupied by 1 mm through image viewing software, and then evaluating a length occupied by a single pixel.

Alternatively, a digestive-tract endoscopic target identification module is further used for calling a pre-trained digestive-tract endoscopic target detection neural network based on multi-scale fusion Transformer to identify a first digestive-tract endoscopic target frozen area of the first frozen image and a second digestive-tract endoscopic target frozen area of the second frozen image, and carrying out key-point detection on the first digestive-tract endoscopic target frozen area and the second digestive-tract endoscopic target frozen area to obtain a first digestive-tract endoscopic target frozen area and a second digestive-tract endoscopic target frozen area after distortion-elimination alignment; and detecting the first long-axis distance according to the first digestive-tract endoscopic target frozen area after the distortion-elimination alignment and detecting the second long-axis distance according to the second digestive-tract endoscopic target frozen area after the distortion-elimination alignment.

Alternatively, the endoscope withdrawing distance is decided by the length occupied by the single pixel.

Alternatively, the endoscopic target structure evaluation system further includes a client module;

the client module is used for carrying out endoscope withdrawing operation prompting in the case that the digestive-tract endoscopic target identification module identifies the digestive-tract endoscopic target structure from the endoscopic examination images; and

the digestive-tract endoscopic target measurement module is further used for generating an auxiliary location frame in an image processing manner, and triggering the client module to draw and display the auxiliary location frame to carry out operation prompting of moving the digestive-tract endoscopic target structure into the auxiliary location frame.

Alternatively, while identifying the digestive-tract endoscopic target structure from the endoscopic examination images, the digestive-tract endoscopic target identification module is specifically used for calling a pre-trained digestive-tract endoscopic target detection neural network based on multi-scale fusion Transformer to identify the digestive-tract endoscopic target structure from the endoscopic examination images.

Alternatively, the digestive-tract endoscopic target detection neural network based on multi-scale fusion Transformer includes a main feature extraction part, a feature fusion part, and a prediction head part;

the main feature extraction part includes a normalization layer, a first feature extraction module formed through connecting three groups of standard residual convolution blocks in series, a second feature extraction module formed through connecting six groups of standard residual convolution blocks in series, a third feature extraction module formed through connecting nine groups of standard residual convolution blocks in series, and a fourth feature extraction module formed through connecting a spatial pyramid pooling module and a Transformer module in series;
the normalization layer is used for zooming the endoscopic examination images according to a pre-set zooming size; the first feature extraction module is used for outputting a first feature image through computing the zoomed endoscopic examination images; the second feature extraction module is used for outputting a second feature image through computing the first feature image; the third feature extraction module is used for outputting a third feature image through computing the second feature image; and the fourth feature extraction module is used for obtaining a fourth feature image through computing the third feature image;
the feature fusion part is mainly used for carrying out attention weighting on the first feature image, the second feature image, the third feature image, and the fourth feature image to respectively obtain a first attention-weighted feature image, a second attention-weighted feature image, a third attention-weighted feature image and a fourth attention-weighted feature image; using a first Transformer module for calculating the first attention-weighted feature image to obtain a first fused feature image; using a second Transformer module for calculating the second attention-weighted feature image and splicing with the first attention-weighted feature image to obtain a second fused feature image; using a third Transformer module for calculating the third attention-weighted feature image and splicing with the second attention-weighted feature image to obtain a third fused feature image; and using a fourth Transformer module for calculating the fourth attention-weighted feature image and splicing with the third attention-weighted feature image to obtain a fourth fused feature image; and
the prediction head part is mainly used for carrying out grid-search prediction on the first fused feature image, the second fused feature image, the third fused feature image, and the fourth fused feature image by means of a pre-set prior frame, and identifying the digestive-tract endoscopic target structure.

In a second aspect, the present invention provides an endoscopic target structure evaluation method, and the endoscopic target structure evaluation method includes:

carrying out endoscope withdrawing operation prompting in the case that a digestive-tract endoscopic target structure is identified from endoscopic examination images;

respectively freezing images of the digestive-tract endoscopic target structure from the endoscopic examination images before an endoscope withdrawing operation and the endoscopic examination images after the endoscope withdrawing operation to obtain a first frozen image and a second frozen image; and

determining a length of the digestive-tract endoscopic target structure according to a first long-axis distance of the first frozen image, a second long-axis distance of the second frozen image, an endoscope withdrawing distance, a length occupied by a single pixel, and a number of pixel points at the first long-axis distance.

In a third aspect, the present invention provides an electronic device, and the electronic device includes: a memory, a controller, and a computer program stored in the memory and capable of being run in the controller, where

the steps of the above endoscopic target structure evaluation method are realized when the computer program is executed by the controller.

In a fourth aspect, the present invention provides a computer-readable storage medium in which an endoscopic target structure evaluation program is stored, where the steps of the above endoscopic target structure evaluation method are realized when the endoscopic target structure evaluation program is executed by the controller.

According to the examples of the present invention, the images of the digestive-tract endoscopic target structure are respectively frozen from the endoscopic examination images before the endoscope withdrawing operation and the endoscopic examination images after the endoscope withdrawing operation to obtain the first frozen image and the second frozen image; and the length of the digestive-tract endoscopic target structure is determined according to the first long-axis distance of the first frozen image, the second long-axis distance of the second frozen image, the endoscope withdrawing distance, the length occupied by the single pixel, and the number of the pixel points at the first long-axis distance. Therefore, problems existing in a visual inspection method and a comparison measurement method with biopsy forceps are effectively solved, so that increasing for accuracy of estimating a lesion size by a doctor is contributed; and a unified standard is established, so that a problem of missed diagnosis for lesions through routine endoscopic visual examination is further effectively solved, and a problem of incapability of accurately measuring a lesion size under an endoscope is avoided.

BRIEF DESCRIPTION OF THE SEVERAL VIEWS OF THE DRAWINGS

FIG. 1 is a block diagram of an endoscopic target structure evaluation system according to an example of the present invention; and

FIG. 2 is a flow diagram of an endoscopic target structure evaluation method according to the example of the present invention.

DETAILED DESCRIPTION OF THE INVENTION

The present invention will be further described below in detail in conjunction with the drawings and specific examples, and it should be understood that, the specific examples described herein are merely used for explaining the present invention and are not intended to limit the present invention.

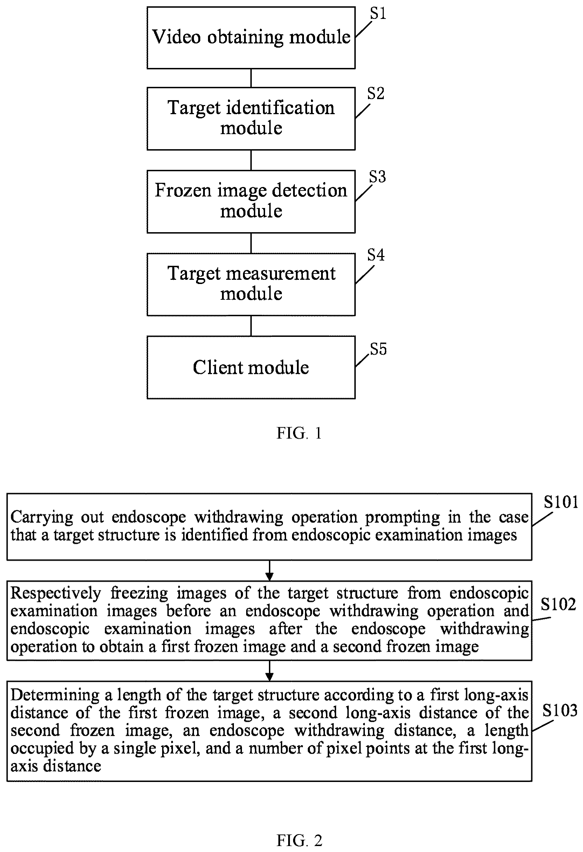

Example 1: the example of the present invention provides an endoscopic target structure evaluation system, and as shown in FIG. 1, the endoscopic target structure evaluation system includes:

the client module is used for carrying out endoscope withdrawing operation prompting in the case that the digestive-tract endoscopic target identification module identifies the digestive-tract endoscopic target structure from the endoscopic examination images; and

a frozen image detection module, which is used for respectively freezing images of the digestive-tract endoscopic target structure from the endoscopic examination images before an endoscope withdrawing operation and the endoscopic examination images after the endoscope withdrawing operation to respectively obtain a first frozen image and a second frozen image; and

a digestive-tract endoscopic target measurement module, which is used for determining a length of the digestive-tract endoscopic target structure according to a first long-axis distance of the first frozen image, a second long-axis distance of the second frozen image, an endoscope withdrawing distance, a length occupied by a single pixel, and a number of pixel points at the first long-axis distance.

The endoscopic target structure evaluation system in the example of the present invention includes the client module, the frozen image detection module, and the digestive-tract endoscopic target measurement module, where the endoscope withdrawing operation prompting is carried out through the client module in the case that the digestive-tract endoscopic target identification module identifies the digestive-tract endoscopic target structure from the endoscopic examination images; the frozen image detection module respectively freezes the images of the digestive-tract endoscopic target structure from the endoscopic examination images before the endoscope withdrawing operation and the endoscopic examination images after the endoscope withdrawing operation to respectively obtain the first frozen image and the second frozen image; and the digestive-tract endoscopic target measurement module determines the length of the digestive-tract endoscopic target structure according to the first long-axis distance of the first frozen image, the second long-axis distance of the second frozen image, the endoscope withdrawing distance, the length occupied by the single pixel, and the number of pixel points at the first long-axis distance. Therefore, problems existing in a visual inspection method and a comparison measurement method with biopsy forceps are effectively solved, so that increasing for accuracy of estimating a lesion size by a doctor is contributed; and a unified standard is established, so that a problem of missed diagnosis for lesions through routine endoscopic visual examination is further effectively solved, and a problem of incapability of accurately measuring a lesion size under an endoscope is avoided.

In some implementation manners, the endoscopic target structure evaluation system may further include a video obtaining module, and detailedly, the endoscopic target structure evaluation system, which is provided by the specific implementation manner includes:

1. S1-a video obtaining module
which is used for reading video signals output by an endoscopic device to obtain video frame images. Specifically, a video capture card may be utilized to transmit digital/analog video signals such as HDMI, DVI, SDI and S-Video into a computer, and the video signals are read through opencv and transcoded into an RGB image format frame by frame to obtain endoscopic examination images.

2. S2-a digestive-tract endoscopic target identification module
which is mainly used for identifying a digestive-tract endoscopic target structure from the endoscopic examination images, and calling a pre-trained digestive-tract endoscopic target detection neural network based on multi-scale fusion Transformer to identify a first digestive-tract endoscopic target frozen area of the first frozen image and a second digestive-tract endoscopic target frozen area of the second frozen image, carrying out key-point detection on the first digestive-tract endoscopic target frozen area and the second digestive-tract endoscopic target frozen area to obtain a first digestive-tract endoscopic target frozen area and a second digestive-tract endoscopic target frozen area after distortion-elimination alignment; and detecting the first long-axis distance according to the first digestive-tract endoscopic target frozen area after the distortion-elimination alignment and detecting the second long-axis distance according to the second digestive-tract endoscopic target frozen area after the distortion-elimination alignment. Detailedly:

(1) an artificial-intelligence digestive-tract endoscopic target detection neural network is constructed, and in the example of the present invention, a digestive-tract endoscopic target detection neural network based on multi-scale fusion Transformer is adopted.

i. A digestive-tract endoscopic target detection neural network architecture is constructed, and includes a main feature extraction part, a feature fusion part, and a prediction head part.

ii. Firstly, the main feature extraction part uses a cross-stage convolutional neural network structure, and includes a normalization layer, a first feature extraction module formed through connecting three groups of standard residual convolution blocks in series, a second feature extraction module formed through connecting six groups of standard residual convolution blocks in series, a third feature extraction module formed through connecting nine groups of standard residual convolution blocks in series, and a fourth feature extraction module formed through connecting a spatial pyramid pooling module and a Transformer module in series; and

specifically, structural functions of the main feature extraction part include that:

1. the normalization layer is used for zooming endoscopic examination images according to a pre-set zooming size, such as zooming input sizes of the images to 640*640;

2. the first feature extraction module is formed through internally connecting three groups of standard residual convolution blocks in series, and outputs a first feature image through computation;

3. the second feature extraction module is formed through internally connecting six groups of standard residual convolution blocks in series, and outputs a second feature image through computation;

4. the third feature extraction module is formed through internally connecting nine groups of standard residual convolution blocks in series, and outputs a third feature image through computation; and

5. the fourth feature extraction module is formed through internally connecting a spatial pyramid pooling module and a Transformer module in series, and used for enhancing explicit advanced features in the third feature image and obtaining a fourth feature image through computation.

iii. Secondly, a main functional structure of the feature fusion part includes that:

1. attention weighting is carried out on the first feature image, the second feature image, the third feature image, and the fourth feature image, and specifically, three groups of residual convolution computations are carried out at first, and then spatial attention weighting is carried out (public spatial attention modules such as a CBAM, an ECA, an RFB and a BAM may be used), and in the example of the present invention, the ECA module is selected to complete spatial attention weighting to obtain a first attention-weighted feature image, a second attention-weighted feature image, a third attention-weighted feature image, and a fourth attention-weighted feature image;

2. a first Transformer module is used for calculating the first attention-weighted feature image to obtain a first fused feature image;

3. a second Transformer module is used for calculating the second attention-weighted feature image and splicing with the first attention-weighted feature image to obtain a second fused feature image;

4. a third Transformer module is used for calculating the third attention-weighted feature image and splicing with the second attention-weighted feature image to obtain a third fused feature image; and

5. a fourth Transformer module is used for calculating the fourth attention-weighted feature image and splicing with the third attention-weighted feature image to obtain a fourth fused feature image.

iv. Finally, a main functional structure of the prediction head part is that:

1. grid-search prediction is carried out on the first fused feature image, the second fused feature image, the third fused feature image, and the fourth fused feature image by means of a pre-set prior frame, the digestive-tract endoscopic target structure is identified, and a prediction result includes center point coordinates, a length, a width, class id, and class confidence; and

2. duplicate result elimination is carried out on an output predicted structure by means of a non-maximum suppression technology, and network-predicted center point coordinates, length, width, class id, and class confidence of the digestive-tract endoscopic target are finally obtained.

(2) A digestive-tract endoscopic target detection neural network is trained to enable a network model to have capability of identifying the digestive-tract endoscopic target.

i. An annotated data set for the digestive-tract endoscopic target is constructed, the network model is trained according to a pre-set data set, and types include: a protruded digestive-tract endoscopic target, a flat digestive-tract endoscopic target, and a recessed digestive-tract endoscopic target.

ii. Images of the data set are input into the network model in batches, errors between network model output and true values of data are calculated by means of a cross entropy loss, and parameters are updated according to back-propagation.

iii. The above steps are repeated until model precision reaches a pre-set value or the training is stopped when a loss value reaches 1e-5, and the pre-constructed artificial-intelligence digestive-tract endoscopic target detection neural network is obtained.

c) The above trained digestive-tract endoscopic target detection neural network model is utilized to identify obtained frame-by-frame images, and finally, an identification result is obtained through prediction of a prediction layer. The identification result is pushed to a client in a coordinate form and by means of a websocket technology, and a rectangular identification frame is drawn by means of a canvas drawing technology.

3. S3-a frozen image detection module, which is used for respectively freezing images of the digestive-tract endoscopic target structure from endoscopic examination images before an endoscope withdrawing operation and endoscopic examination images after the endoscope withdrawing operation to obtain a first frozen image and a second frozen image; and detailedly:

   a) when the identification result for the digestive-tract endoscopic target occurs in a video frame image, a user is prompted to start a digestive-tract endoscopic target measurement function; and a client module is utilized to display an auxiliary location frame at the center of the frame image, and the operating user is guided to move the digestive-tract endoscopic target into the auxiliary location frame to carry out image freezing;

   b) an obtained frame image sequence is identified in a matrix comparison manner to obtain a first frozen image, and according to the identification result for the digestive-tract endoscopic target, first digestive-tract endoscopic target detection coordinates, that is, a first digestive-tract endoscopic target frozen area, is obtained; and

   c) after the first frozen image is identified, the client module is utilized to prompt the user to freeze the image again after a first-time endoscope withdrawing centimeter (0.5 cm in the example of the present invention); and the obtained frame image sequence is identified in the matrix comparison manner to obtain a second frozen image, and according to the identification result for the digestive-tract endoscopic target, second digestive-tract endoscopic target detection coordinates, that is, a second digestive-tract endoscopic target frozen area, is obtained.

4. S4-a digestive-tract endoscopic target measurement module, which is used for determining a length of the digestive-tract endoscopic target structure according to a first long-axis distance of the first frozen image, a second long-axis distance of the second frozen image, an endoscope withdrawing distance, a length occupied by a single pixel, and a number of pixel points at the first long-axis distance, and detailedly:

   a) an auxiliary location frame is automatically generated by means of an image processing technology, and the operating user is guided to move the digestive-tract endoscopic target into the auxiliary location frame to carry out image freezing;

   b) when a frozen image detection module detects freezing, and meanwhile, the digestive-tract endoscopic target identification module obtains the identification result, the first frozen image is automatically stored, and according to a prediction result of the digestive-tract endoscopic target identification module, first digestive-tract endoscopic target detection coordinates are obtained;

   c) after the first frozen image is identified, the client module is utilized to prompt the user to freeze the image again after a first-time endoscope withdrawing centimeter (in the example, the endoscope withdrawing distance is decided by the length occupied by the single pixel, for example, in the case of 1 cm away from a caliper during measurement, then $\delta$ is a pixel proportion under 1 cm diagnosis, and the endoscope withdrawing L must also be 1 cm; and if a measurer is 2 cm away from the caliper, an endoscope withdrawing range may be 0.4 to 1 cm, and preferably 1 cm); and the frozen image detection module is utilized to obtain a second frozen image, and according to a prediction result of the digestive-tract endoscopic target identification module, second digestive-tract endoscopic target detection coordinates are obtained;

   d) a digestive-tract endoscopic target distortion of the digestive-tract endoscopic target, which is brought by the "endoscope withdrawing centimeter" operation is eliminated, and specifically, key-point extraction and matching are carried out on areas of the first digestive-tract endoscopic target detection coordinates and the second digestive-tract endoscopic target detection coordinates by means of an OBR key-point detection technology, RANSAC coarse error elimination is executed on matched key points, and then perspective transformation is carried out by means of the matched key points to obtain first digestive-tract endoscopic target coordinates and second digestive-tract endoscopic target coordinates after distortion-elimination alignment;

   e) long-axis distances of the first digestive-tract endoscopic target coordinates and the second digestive-tract endoscopic target coordinates are detected by means of a convex hull algorithm, and a first long-axis distance $B_1$ and a second long-axis distance $B_2$ are obtained; and

   f) a length $B$ of the digestive-tract endoscopic target is calculated by means of a conversion formula. The conversion formula is as follows:

$$B=\frac{B_2 \times L}{B_2 - B_1} \times \vartheta \times Pixs(B_1)$$

where $B_1$ represents a first long-axis distance of the first frozen image, $B_2$ represents a second long-axis distance of the second frozen image, $L$ represents an endoscope withdrawing distance, $\vartheta$ represents a length occupied by a single pixel, and $Pixs(B_1)$ represents a number of pixel points at the first long-axis distance.

5. S5-the client module, which is used for:

a) carrying out endoscope withdrawing operation prompting in the case that the digestive-tract endoscopic target identification module identifies the digestive-tract endoscopic target structure from the endoscopic examination images;

b) drawing a detection frame in a video frame image according to an identification result of the digestive-tract endoscopic target identification module;

c) drawing an auxiliary location frame for the digestive-tract endoscopic target according to the digestive-tract endoscopic target measurement module; and

d) displaying a size of the digestive-tract endoscopic target according to a detection result of the digestive-tract endoscopic target measurement module.

The implementation manner discloses a digestive-tract endoscopic target structure measurement method, which is particularly applicable for polyp measurement, and a purpose of accurate measurement for lesions in clinical practice is achieved by means of a plurality of combination technologies such as a pixel point mapping length of an endoscopic measurement lens, freezing detection, artificial-intelligence digestive-tract endoscopic target detection, and a length conversion formula. In addition, the digestive-tract endoscopic target detection network based on multi-scale fusion Transformer has four scales of fusion, and meanwhile, due to the utilization for dual support of Transformer and spatial attention, capability of capturing the digestive-tract endoscopic target is greatly improved, and meanwhile, occurrence of false positives is also reduced. Problems existing in a visual inspection method and a comparison measurement method with biopsy forceps are effectively solved, so that increasing for accuracy of estimating a size of a digestive-tract endoscopic target by a doctor is contributed; and a unified standard is established, so that a problem of missed diagnosis for the digestive-tract endoscopic target through routine endoscopic visual examination is further effectively solved, and a problem of incapability of accurately measuring the size of the digestive-tract endoscopic target under an endoscope is avoided.

Example 2: the example of the present invention provides an endoscopic target structure evaluation method, and as shown in FIG. 2, the endoscopic target structure evaluation method includes:

S101, carrying out endoscope withdrawing operation prompting in the case that a digestive-tract endoscopic target structure is identified from endoscopic examination images;

S102, respectively freezing images of a digestive-tract endoscopic target structure from the endoscopic examination images before an endoscope withdrawing operation and the endoscopic examination images after the endoscope withdrawing operation to obtain a first frozen image and a second frozen image; and

S103, determining a length of the digestive-tract endoscopic target structure according to a first long-axis distance of the first frozen image, a second long-axis distance of the second frozen image, an endoscope withdrawing distance, a length occupied by a single pixel, and a number of pixel points at the first long-axis distance.

In some implementation manners, a length $B$ of the digestive-tract endoscopic target structure is determined through adopting a following formula:

$$B=\frac{B_2 \times L}{B_2 - B_1} \times \vartheta \times Pixs(B_1)$$

where $B_1$ represents a first long-axis distance of the first frozen image, $B_2$ represents a second long-axis distance of the second frozen image, $L$ represents an endoscope withdrawing distance, $\vartheta$ represents a length occupied by a single pixel, and $Pixs(B_1)$ represents a number of pixel points at the first long-axis distance.

In some implementation manners, a pre-trained digestive-tract endoscopic target detection neural network based on multi-scale fusion Transformer is called to identify a first digestive-tract endoscopic target frozen area of the first frozen image and a second digestive-tract endoscopic target frozen area of the second frozen image, and key-point detection is carried out on the first digestive-tract endoscopic target frozen area and the second digestive-tract endoscopic target frozen area to obtain a first digestive-tract endoscopic target frozen area and a second digestive-tract endoscopic target frozen area after distortion-elimination alignment; and the first long-axis distance is detected according to the first digestive-tract endoscopic target frozen area after the distortion-elimination alignment and the second long-axis distance is detected according to the second digestive-tract endoscopic target frozen area after the distortion-elimination alignment.

Alternatively, an endoscope withdrawing distance range is 0.4 to 1 centimeter.

In some implementation manners, the endoscopic target structure evaluation method further includes: generating an auxiliary location frame in an image processing manner, and triggering the client module to draw and display the auxiliary location frame to carry out operation prompting of moving the digestive-tract endoscopic target structure into the auxiliary location frame.

In some implementation manners, the identifying the digestive-tract endoscopic target structure from endoscopic examination images includes: a pre-trained digestive-tract endoscopic target detection neural network based on multi-scale fusion Transformer identifies the digestive-tract endoscopic target structure from endoscopic examination images.

Example 3: the example of the present invention provides an electronic device, and the electronic device includes: a memory, a controller, and a computer program stored in the memory and capable of being run in the controller, where

the steps of the endoscopic target structure evaluation method in the example 2 are realized when the computer program is executed by the controller.

Example 4: the example of the present invention provides a computer-readable storage medium in which an endoscopic target structure evaluation program is stored, where the steps of the endoscopic target structure evaluation method in the example 2 are realized when the endoscopic target structure evaluation program is executed by a controller.

The example 1 may be referred to for the examples 2 to 4 in specific realization processes, and corresponding technical effects are achieved.

The examples of the present invention have been described above in conjunction with the drawings, however, the present invention is not limited to the above specific implementation manners, the above specific implementation manners are merely illustrative and not restrictive, and those of ordinary skill in the art may make many forms under the inspiration of the present invention without departing from the purpose and the protection scope of the claims of the present invention, and these forms are all within the protection of the present invention.

## Claims

1. An endoscopic target structure evaluation system, comprising:

a frozen image detection module, which is used for respectively freezing images of a digestive-tract endoscopic target structure from endoscopic examination images before an endoscope withdrawing operation and endoscopic examination images after the endoscope withdrawing operation to respectively obtain a first frozen image and a second frozen image; and a digestive-tract endoscopic target measurement module, which is used for determining a length of the digestive-tract endoscopic target structure according to a first long-axis distance of the first frozen image, a second long-axis distance of the second frozen image, an endoscope withdrawing distance, a length occupied by a single pixel, and a number of pixel points at the first long-axis distance.

2. The endoscopic target structure evaluation system according to claim 1, **characterized in that** the digestive-tract endoscopic target measurement module determines a length $B$ of the digestive-tract endoscopic target structure through adopting a following formula:

$$B = \frac{B_2 \times L}{B_2 - B_1} \times \vartheta \times Pixs(B_1)$$

wherein $B_1$ represents a first long-axis distance of the first frozen image, $B_2$ represents a second long-axis distance of the second frozen image, $L$ represents an endoscope withdrawing distance, $\vartheta$ represents a length occupied by a single pixel, and $Pixs(B_1)$ represents a number of pixel points at the first long-axis distance.

3. The endoscopic target structure evaluation system according to claim 2, **characterized in that** the endoscope withdrawing distance is decided by the length occupied by the single pixel.

4. The endoscopic target structure evaluation system according to claim 1, **characterized in that** a digestive-tract endoscopic target identification module is further used for calling a pre-trained digestive-tract endoscopic target detection neural network based on multi-scale fusion Transformer to identify a first digestive-tract endoscopic target frozen area of the first frozen image and a second digestive-tract endoscopic target frozen area of the second frozen image, carrying out key-point detection on the first digestive-tract endoscopic target frozen area and the second digestive-tract endoscopic target frozen area to obtain a first digestive-tract endoscopic target frozen area and a second digestive-tract endoscopic target frozen area after distortion-elimination alignment; and detecting the first long-axis distance according to the first digestive-tract endoscopic target frozen area after the distortion-elimination alignment and detecting the second long-axis distance according to the second digestive-tract endoscopic target frozen area after the distortion-elimination alignment.

5. The endoscopic target structure evaluation system according to claim 1, further comprising a client module, **characterized in that**

the client module is used for carrying out endoscope withdrawing operation prompting in the case that the digestive-tract endoscopic target identification module identifies the digestive-tract endoscopic target structure from the endoscopic examination images; and
the digestive-tract endoscopic target measurement module is further used for generating an auxiliary location frame in an image processing manner, and triggering the client module to draw and display the auxiliary location frame to carry out operation prompting of moving the digestive-tract endoscopic target structure into the auxiliary location frame.

6. The endoscopic target structure evaluation system according to any one of claims 1 to 5, **characterized in that** while identifying the digestive-tract endoscopic target structure from the endoscopic examination images, the digestive-tract endoscopic target identification module is specifically used for calling a pre-trained digestive-tract endoscopic target detection neural network based on multi-scale fusion Transformer to identify the digestive-tract endoscopic target structure from the endoscopic examination images.

7. The endoscopic target structure evaluation system according to claim 6, **characterized in that** the digestive-tract endoscopic target detection neural network based on multi-scale fusion Transformer comprises a main feature extraction part, a feature fusion part, and a prediction head part;

the main feature extraction part comprises a normalization layer, a first feature extraction module formed through connecting three groups of standard residual convolution blocks in series, a second feature extraction module formed through connecting six groups of standard residual convolution blocks in series, a third feature extraction module formed through connecting nine groups of standard residual convolution blocks in series, and a fourth feature extraction module formed through connecting a spatial pyramid pooling module and a Transformer module in series;
the normalization layer is used for zooming the endoscopic examination images according to a pre-set zooming size; the first feature extraction module is used for outputting a first feature image through computing the zoomed endoscopic examination images; the second feature extraction module is used for outputting a second feature image through computing the first feature image; the third feature extraction module is used for outputting a third feature image through computing the second feature image; and the fourth feature extraction module is used for obtaining a fourth feature image through computing the third feature image;
the feature fusion part is mainly used for carrying out attention weighting on the first feature image, the second feature image, the third feature image, and the fourth feature image to respectively obtain a first attention-weighted feature image, a second attention-weighted feature image, a third attention-weighted feature image and a fourth attention-weighted feature image; using a first Transformer module for calculating the first attention-weighted feature image to obtain a first fused feature image; using a second Transformer module for calculating the second attention-weighted feature image and splicing with the first attention-weighted feature image to obtain a second fused feature image; using a third Transformer module for calculating the third attention-weighted feature image and splicing with the second attention-weighted feature image to obtain a third fused feature image; and using a fourth Transformer module for calculating the fourth attention-weighted feature image and splicing with the third attention-weighted feature image to obtain a fourth fused feature image; and
the prediction head part is mainly used for carrying out grid-search prediction on the first fused feature image, the second fused feature image, the third fused feature image, and the fourth fused feature image by means of a pre-set prior frame, and identifying the digestive-tract endoscopic target structure.

8. An endoscopic target structure evaluation method, comprising:

carrying out endoscope withdrawing operation prompting in the case that a digestive-tract endoscopic target structure is identified from endoscopic examination images;
respectively freezing images of the digestive-tract endoscopic target structure from the endoscopic examination images before an endoscope withdrawing operation and the endoscopic examination images after the endoscope withdrawing operation to obtain a first frozen image and a second frozen image; and
determining a length of the digestive-tract endoscopic target structure according to a first long-axis distance of the first frozen image, a second long-axis distance of the second frozen image, an endoscope withdrawing distance, a length occupied by a single pixel, and a number of pixel points at the first long-axis distance.

**9.** An electronic device, comprising: a memory, a controller, and a computer program stored in the memory and capable of being run in the controller, wherein

the steps of the endoscopic target structure evaluation method according to claim 8 are realized when the computer program is executed by the controller.

**10.** A computer-readable storage medium in which an endoscopic target structure evaluation program is stored, wherein the steps of the endoscopic target structure evaluation method according to claim 8 are realized when the endoscopic target structure evaluation program is executed by a controller.

**11**

Video obtaining module /S1

Target identification module /S2

Frozen image detection module /S3

Target measurement module /S4

Client module /S5

FIG. 1

Carrying out endoscope withdrawing operation prompting in the case that a target structure is identified from endoscopic examination images /S101

Respectively freezing images of the target structure from endoscopic examination images before an endoscope withdrawing operation and endoscopic examination images after the endoscope withdrawing operation to obtain a first frozen image and a second frozen image /S102

Determining a length of the target structure according to a first long-axis distance of the first frozen image, a second long-axis distance of the second frozen image, an endoscope withdrawing distance, a length occupied by a single pixel, and a number of pixel points at the first long-axis distance /S103

FIG. 2

## INTERNATIONAL SEARCH REPORT

| International application No. |
|---|
| **PCT/CN2024/075039** |

| A. | CLASSIFICATION OF SUBJECT MATTER |
|---|---|

G06T 7/00(2017.01)i; G06V 10/774(2022.01)i; G06V 10/82(2022.01)i; G06V 10/80(2022.01)i; G06T 3/40(2024.01)i; G06V 20/40(2022.01)i

According to International Patent Classification (IPC) or to both national classification and IPC

| B. | FIELDS SEARCHED |
|---|---|

Minimum documentation searched (classification system followed by classification symbols)

IPC: G06T G06V

Documentation searched other than minimum documentation to the extent that such documents are included in the fields searched

Electronic data base consulted during the international search (name of data base and, where practicable, search terms used)

CNABS; CNTXT; CNKI; VEN; USTXT; WOTXT; EPTXT; IEEE: 内窥镜, 轴距, 长度, 大小, 尺寸, 退镜, 距离, 特征图, 第一, 第二, 第三, 第四, 畸变, 预测头, endoscope, wheelbase, length, size, retraction, distance, feature map, first, second, third, fourth, distortion, prediction head

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 115797348 A (TIANJIN YUJIN ARTIFICIAL INTELLIGENCE MEDICAL TECHNOLOGY CO., LTD.) 14 March 2023 (2023-03-14) claims 1-10, and description, paragraphs [0020]-[0066] | 1-10 |
| X | CN 113240726 A (NANKAI UNIVERSITY) 10 August 2021 (2021-08-10) description, paragraphs [0039]-[0062] | 1-3, 5, 8 |
| Y | CN 113240726 A (NANKAI UNIVERSITY) 10 August 2021 (2021-08-10) description, paragraphs [0039]-[0062] | 4, 6, 7, 9, 10 |
| Y | CN 114419381 A (URBAN CLOUD TECHNOLOGY (CHINA) LIMITED COMPANY) 29 April 2022 (2022-04-29) description, paragraphs [0028]-[0079] | 4, 6, 7, 9, 10 |
| A | CN 111739007 A (SOUTH-CENTRAL UNIVERSITY FOR NATIONALITIES) 02 October 2020 (2020-10-02) entire document | 1-10 |

☐ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **14 March 2024** | **28 March 2024** |

| Name and mailing address of the ISA/CN | Authorized officer |
|---|---|
| **China National Intellectual Property Administration (ISA/ CN)** <br> **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

International application No.

**PCT/CN2024/075039**

| Patent document cited in search report | | | Publication date (day/month/year) | Patent family member(s) | | | Publication date (day/month/year) |
|---|---|---|---|---|---|---|---|
| CN | 115797348 | A | 14 March 2023 | CN | 115797348 | B | 28 April 2023 |
| | | | | HK | 40083937 | A0 | 07 July 2023 |
| | | | | HK | 40083937 | A1 | 06 October 2023 |
| CN | 113240726 | A | 10 August 2021 | CN | 113240726 | B | 14 October 2022 |
| CN | 114419381 | A | 29 April 2022 | CN | 114419381 | B | 24 June 2022 |
| CN | 111739007 | A | 02 October 2020 | | None | | |

Form PCT/ISA/210 (patent family annex) (July 2022)

**EP 4 664 397 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310069871